# FASCICULE DE BREVET EUROPEEN

(11) **EP 1 018 482 B1**
(45) Date de publication et mention de la délivrance du brevet: **02.07.2003**
(21) Numéro de dépôt: 99403315.7
(22) Date de dépôt: 29.12.1999
(51) Int. Cl.: B67D 5/02

(54) **Procédé et dispositif de transfert de produits stériles entre conteneurs**
Verfahren und Vorrichtung zum Umfüllen von sterilen Produkten zwischen Behältern
Method and device for transferring sterile products between containers

(30) Priorité: 04.01.1999 FR 9900014
(43) Date de publication de la demande: 12.07.2000
(73) Titulaire: La Calhene, 78140 Vélizy Villacoublay (FR)
(72) Inventeur: Brossard, Jean-Pierre, 78400 Chatou (FR); Fontcuberta, Philippe, 41100 Vendome (FR); Riviere, Jean-Michel, 41100 Villeromain (FR)
(74) Mandataire: Poulin, Gérard

(56) Documents cités:
- WO-A-96/21615
- FR-A- 2 658 489
- US-A- 5 490 546

## Description

### Domaine technique

L'invention concerne un procédé et un dispositif permettant de transférer dans un isolateur stérile des produits préalablement stérilisés, contenus dans un conteneur de transport.

L'invention peut être utilisée dans tous les domaines industriels impliquant la manutention de produits stériles, et notamment dans les industries pharmaceutique, médicale, chimique, alimentaire, etc..

Dans l'ensemble du texte, le terme "produits" doit être pris dans son sens le plus large, c'est-à-dire qu'il couvre notamment des poudres, des granulés, des composants de forme et de nature diverses, des liquides, etc..

### Etat de la technique

Pour assurer le transport de produits préalablement stérilisés, par exemple par irradiation gamma, jusqu'à un isolateur dans lequel ces produits doivent subir des opérations quelconques, par exemple de conditionnement, il est connu de placer ces produits dans des conteneurs en forme de sacs souples.

Le raccordement d'un conteneur sur l'isolateur et le transfert des produits à l'intérieur de ce dernier sont notamment effectués au moyen dispositifs de raccordement à double porte assurant un transfert étanche. Un tel dispositif comprend, de façon connue, une bride normalement obturée par une porte sur le conteneur et une bride normalement obturée par une porte sur l'isolateur. Lors de l'accostage du conteneur sur l'isolateur, les deux brides sont fixées l'une à l'autre de manière étanche et les deux portes sont fixées l'une à l'autre de manière étanche. Après ouverture de la double porte ainsi formée, depuis l'intérieur de l'isolateur, le transfert des produits peut être effectué sans qu'il soit nécessaire de procéder à une quelconque opération de stérilisation préalable.

Cette technique connue est sûre et généralement satisfaisante. Lorsque la fréquence des transferts est importante, elle présente toutefois comme inconvénient d'être coûteuse. En effet, après chaque transfert, les conteneurs doivent être soit jetés, soit recyclés.

Compte tenu du prix de l'ensemble bride/porte qui équipe chaque conteneur, la mise au rebut des conteneurs après leur utilisation conduit à un coût d'exploitation d'autant plus élevé que le nombre de conteneurs utilisés augmente.

Par ailleurs, les opérations de recyclage des conteneurs sont elles aussi coûteuses et pénalisantes dès que le nombre de conteneurs utilisés s'accroît. En effet, elles imposent d'emballer les ensembles bride/porte après usage, puis de les déballer, d'en assurer un contrôle visuel et un nettoyage, avant de les remonter et d'en assurer un nouveau contrôle et un nouvel emballage.

Une autre technique connue, décrite notamment dans le document WO-A-96 21615 consiste à équiper chaque conteneur d'une bride fermée par un opercule. Cela permet d'abaisser sensiblement le prix d'un conteneur, de sorte que celui-ci peut être jeté après chaque utilisation. Le coût d'exploitation du système reste ainsi raisonnable lorsque le nombre de transferts augmente.

Du fait de l'abandon du dispositif de transfert à double porte, cette technique à toutefois pour inconvénient qu'un volume non stérile est créé, après le raccordement du conteneur sur l'isolateur, entre l'opercule fermant le conteneur et la porte fermant l'isolateur.

Dans le document WO-A-96 21615, il est proposé de remédier à cet inconvénient en équipant la porte de l'isolateur, sur sa face tournée vers l'extérieur, de moyens de stérilisation constitués par des lampes ultraviolet. Plus précisément, la porte de l'isolateur vient s'appliquer de façon étanche contre la face intérieure de la bride de l'isolateur et l'opercule du conteneur vient se placer pratiquement dans le plan de cette face, lorsque l'accostage est réalisé. Le petit volume clos formé entre l'opercule et la porte est alors stérilisé. Ensuite, la porte de l'isolateur est ouverte et l'opercule est découpé, afin de permettre le transfert des produits.

Ce système présente toutefois quelques inconvénients.

Ainsi, du fait de l'implantation des moyens de stérilisation dans la porte de l'isolateur, les interventions de maintenance (remplacement d'une lampe, réparation électrique, etc.) ne peuvent être effectuées qu'après ouverture de la porte de l'isolateur et depuis l'intérieur de celui-ci. Cela entraîne une rupture d'étanchéité et donc de stérilité de l'isolateur qui ne peut être évitée qu'à la condition de réaliser l'intervention en présence d'un conteneur ou d'un obturateur spécifique prévu à cet effet. De plus, la nécessité d'intervenir depuis l'intérieur de l'isolateur est contraignante.

Par ailleurs, la technique de stérilisation au moyen de lampes ultraviolet est peu performante, ce qui conduite à limiter l'utilisation du système à des ouvertures de transfert de relativement petites dimensions.

### Exposé de l'invention

L'invention a précisément pour objet un procédé de transfert de produits stériles entre un conteneur et un isolateur permettant de remédier aux inconvénients des procédés existants, en autorisant l'utilisation de conteneurs jetables peu coûteux, tout en assurant une maintenance aisée des moyens de stérilisation.

Conformément à l'invention, ce résultat est obtenu au moyen d'un procédé de transfert de produits stériles, entre un conteneur de transport et un isolateur stérile, selon lequel :
- on met en position d'accostage le conteneur par rapport à l'isolateur;
- on stérilise un volume clos délimité entre des obturateurs respectifs du conteneur et de l'isolateur ; et
- on ouvre lesdits obturateurs avant de procéder au transfert des produits ;
caractérisé en ce que en position d'accostage par rapport à l'isolateur, on accoste le conteneur sur un sas raccordé extérieurement à l'isolateur et normalement séparé de celui-ci par son obturateur, ledit sas incluant des moyens de stérilisation et délimitant le volume clos ;
et caractérisé en ce qu'on stérilise le volume clos en mettant en oeuvre des moyens de stérilisation en lumière pulsée placés autour d'un tube transparent délimitant le volume clos.

Cet agencement permet d'utiliser un conteneur jetable peu coûteux, muni d'un simple obturateur. De plus, il autorise des interventions de maintenance rapides et aisées sur les moyens de stérilisation. Enfin, il permet d'obtenir une stérilisation rapide et performante sans limiter les dimensions de l'ouverture de transfert.

Dans un mode de réalisation préféré de l'invention, avant d'accoster le conteneur sur le sas, on raccorde celui-ci sur l'isolateur par l'intermédiaire d'un dispositif de transfert étanche à double porte, dont la double porte forme l'obturateur de l'isolateur. Cet agencement permet, si nécessaire, de remplacer très rapidement les moyens de stérilisation dans leur ensemble.

Avantageusement, on procède au transfert des produits après avoir retourné à l'intérieur du sas une manche de protection interne au conteneur.

On réalise de préférence l'ouverture de l'obturateur du conteneur par découpage depuis l'intérieur de l'isolateur, après ouverture de l'obturateur de celui-ci.

Afin d'améliorer encore l'efficacité de la stérilisation, on peut utiliser un conteneur muni d'un obturateur dont la face extérieure, apte à être tournée vers le volume clos, est réfléchissante.

Selon le cas, le raccordement du conteneur sur le sas peut être assuré soit en utilisant un conteneur souple et en accostant celui-ci sur le sas par emmanchement élastique, soit en utilisant un conteneur présentant une bride semi-rigide portant l'obturateur du conteneur et en accostant celui-ci sur le sas par emboîtement de la bride dans une bride complémentaire du sas.

L'invention a aussi pour objet un dispositif de transfert de produits stériles entre un conteneur de transport et un isolateur stérile, comprenant :
- un obturateur de conteneur ;
- un obturateur d'isolateur ;
- des moyens d'accostage du conteneur sur l'isolateur, aptes à délimiter un volume clos entre l'obturateur de conteneur et l'obturateur d'isolateur, lorsque l'accostage est réalisé ;
- des moyens de stérilisation dudit volume clos ;
le dispositif étant caractérisé en ce qu'il comprend de plus un sas raccordé extérieurement à l'isolateur et normalement séparé de celui-ci par l'obturateur d'isolateur, ledit sas incluant les moyens de stérilisation et comprenant un tube transparent délimitant le volume clos entre les obturateurs de conteneur et d'isolateur, les moyens de stérilisation étant des moyens de stérilisation en lumière pulsée et entourant le tube transparent.

### Brève description des dessins

On décrira à présent, à titre d'exemple non limitatif, un mode de réalisation préféré de l'invention, en se référant aux dessins annexés, dans lesquels :
- la figure 1 est une vue de face, représentant schématiquement, en coupe, un dispositif de transfert selon un mode de réalisation préféré de l'invention ; et
- la figure 2 est une vue en coupe comparable à la figure 1, illustrant une variante du raccordement du conteneur sur le sas.

### Description détaillée d'un mode de réalisation préféré de réalisation

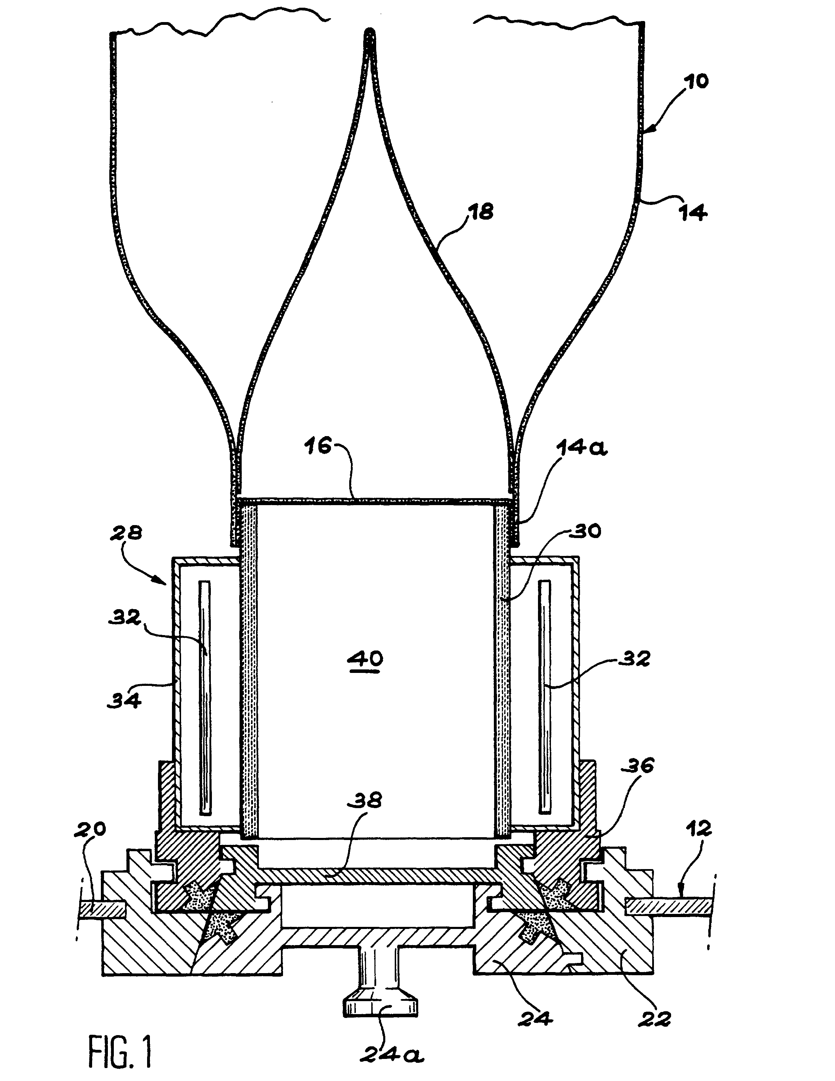

Sur la figure 1, la référence 10 désigne un conteneur de transport destiné à recevoir des produits stériles et la référence 12 désigne un isolateur stérile dans lequel doivent être transférés ces produits (seule la partie de cet isolateur destinée au raccordement d'un conteneur est illustrée).

Le conteneur de transport 10 présente une paroi souple 14 dans laquelle est formée une ouverture, normalement fermée par un obturateur 16. De préférence, l'obturateur 16 se présente sous la forme d'un opercule apte à être découpé afin d'ouvrir le conteneur. Pour une raison qui apparaîtra par la suite, la face de l'obturateur 16 tournée vers l'extérieur du conteneur est, de préférence, rendue réfléchissante, par exemple grâce à une métallisation.

Dans le mode de réalisation représenté sur la figure 1, l'obturateur 16 est un opercule souple dont la partie périphérique est soudée sur une partie tubulaire élastique 14a de la paroi 14, délimitant l'ouverture du conteneur 10.

De plus, une manche de protection 18, également soudée sur la partie 14a de la paroi 14 du conteneur, est normalement placée à l'intérieur de ce dernier. Cette manche de protection, également réalisée dans un matériau souple analogue à celui qui forme la paroi 14, peut être selon le cas ouverte ou fermée.

Par ailleurs, l'isolateur 12 comporte une paroi 20 dans laquelle une ouverture est délimitée par une bride 22. Cette ouverture est normalement fermée par un obturateur 24, constitué par une porte reçue de façon étanche dans la bride 22. Une poignée 24a permet l'ouverture de la porte 24 vers l'intérieur de l'isolateur 12 lorsque des moyens de verrouillage, par exemple à baïonnette (non représentés) sont libérés.

Conformément à l'invention, le conteneur de transport 10 n'est pas accosté directement sur l'isolateur stérile 12, mais sur un sas 28 raccordé extérieurement à l'isolateur 12.

De façon plus précise, le sas 28 comprend un tube transparent 30, réalisé par exemple en quartz, ainsi que des moyens de stérilisation en lumière pulsée matérialisés par des lampes 32 régulièrement réparties autour du tube 30, à l'intérieur d'un carter de protection 34.

Une extrémité du tube 30 est équipée d'une bride 36 délimitant intérieurement une ouverture normalement obturée de façon étanche par une porte 38. La porte 38 coopère avec la bride 36 par des moyens de verrouillage, par exemple à baïonnette.

La bride 36 est prévue pour être raccordée de façon étanche sur la bride 22 et la porte 38 est prévue pour être raccordée de façon étanche sur la porte 24 lorsque le sas 28 est accouplée à l'isolateur 12, comme l'illustre la figure 1. Des moyens de raccordement, par exemple à baïonnette, sont prévus à cet effet entre les brides 22 et 36 et entre les portes 24 et 38. Dans ces conditions, l'ensemble formé par les brides 22 et 36 et par les portes 24 et 38 forme un dispositif de transfert étanche à double porte permettant d'assurer un transfert étanche entre le volume 40 délimité à l'intérieur du tube 30 et l'intérieur de l'isolateur 12, lors de l'ouverture de la double porte formée par les portes 24 et 38 connectées l'une à l'autre.

Lorsque le sas 28 est accouplé à l'isolateur 12, et lorsque le conteneur 10 est accosté sur le sas 28, le volume clos 40 délimité entre la double porte 24, 38 et l'obturateur 16, à l'intérieur du tube 30, est apte à être stérilisé par la mise en oeuvre des moyens de stérilisation 32.

Dans le mode de réalisation illustré sur la figure 1, l'extrémité du tube transparent 30 opposée à la bride 36 se prolonge au-delà du carter 34, de façon à permettre l'emmanchement élastique de la partie tubulaire 14a de la paroi 14 du conteneur 10. Lorsque cet emmanchement est réalisé, la surface extérieure réfléchissante de l'obturateur 16 est tournée vers le volume clos 40 délimité à l'intérieur du sas 28.

Lors de la mise en oeuvre normale du dispositif, le sas 28 reste normalement raccordé sur l'isolateur 12 par l'intermédiaire du dispositif de transfert à double porte constituée par les brides 22 et 36 et par les portes 24 et 38. Lorsqu'un transfert de produits stériles doit être effectué, on accoste un conteneur 10 contenant les produits à transférer sur l'extrémité du tube transparent 30 de la manière qui vient d'être décrite.

Lorsque le raccordement étanche du conteneur est terminé, on met en oeuvre les moyens de stérilisation 32 en lumière pulsée, de façon à stériliser le volume clos 40 ainsi que les surfaces de l'opercule 16, de la porte 38 et du tube 30 qui délimitent ce volume. Il est à noter que l'efficacité de cette stérilisation est améliorée par le caractère réfléchissant de la surface de l'opercule 16 tournée vers le volume 40. On réalise ainsi une stérilisation efficace en moins de 5 mn.

Lorsque la stérilisation est terminée, la double porte formée par les portes 24 et 38 est ouverte depuis l'intérieur de l'isolateur 12. L'opérateur découpe alors l'obturateur 16 depuis l'intérieur de l'isolateur et retourne la manche de protection 18 à l'intérieur du sas 28, de façon à assurer le guidage des produits à transférer jusque dans l'isolateur 12 en évitant tout contact direct des produits avec les surfaces internes du sas et du dispositif de transfert étanche à double porte. Selon le cas, le transfert des produits s'effectue immédiatement si la manche 18 est ouverte, ou après découpe de l'extrémité de cette manche dans le cas contraire.

Lorsque le transfert est terminé, la double porte formée par les portes 24 et 38 est refermée et le conteneur 10 est déconnecté du sas 28, puis jeté.

Grâce à l'agencement conforme à l'invention, qui permet de placer les moyens de stérilisation 32 à l'extérieur de l'isolateur 12, toute intervention sur ces moyens de stérilisation peut être faite sans difficulté depuis l'extérieur de l'isolateur, par exemple pour remplacer l'une des lampes ou pour réparer une liaison électrique.

De plus, dans le cas d'une panne plus sérieuse, la présence du dispositif de transfert à double porte entre le sas 28 et l'isolateur 12 permet de remplacer aisément l'ensemble du sas par un sas équipé de moyens de stérilisation en état de marche, dans un temps particulièrement court et donc d'une manière non pénalisante lorsqu'une cadence importante de transfert de produits est souhaitée.

En outre, l'utilisation de moyens de stérilisation en lumière pulsée assure une stérilisation particulièrement rapide et efficace du volume 40. Cela permet notamment d'assurer un transfert de produits au travers d'une ouverture présentant si nécessaire une section importante. Cela permet également d'accélérer la cadence par rapport à des moyens de stérilisation de type ultraviolet.

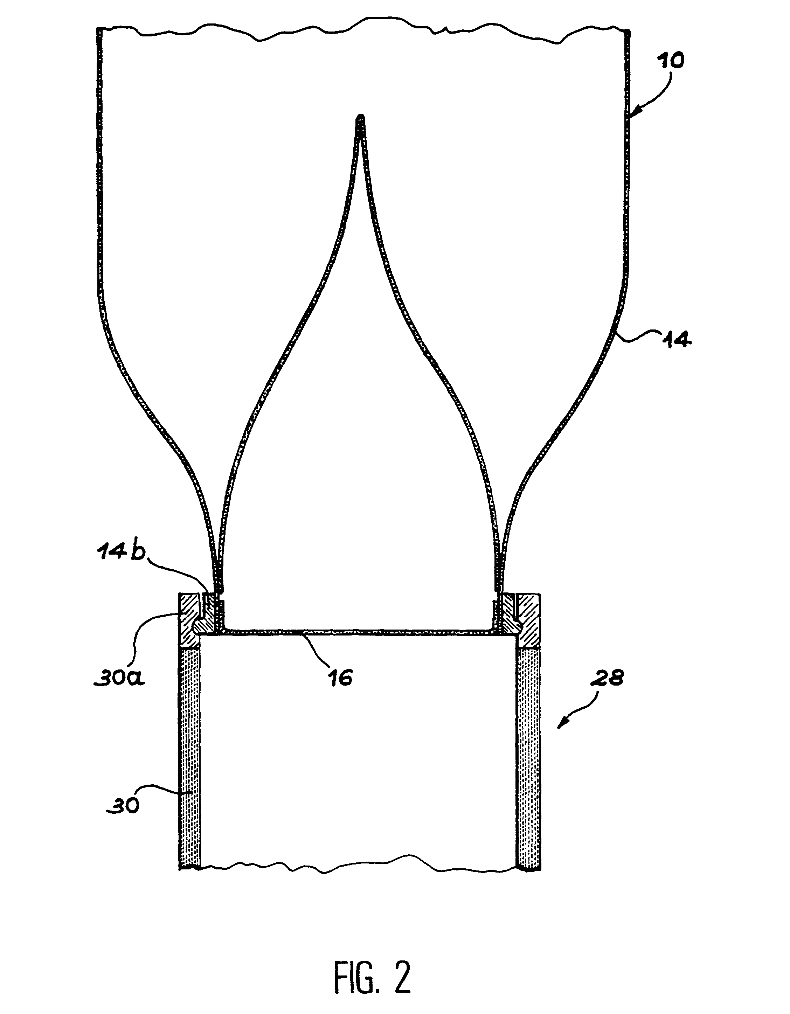

Sur la figure 2, on a représenté schématiquement une variante de réalisation de l'invention. Cette variante diffère essentiellement du mode de réalisation de la figure 1 par les moyens d'accostage du conteneur 10 sur le sas 28.

Ainsi, dans ce cas la partie tubulaire élastique 14a de la paroi 14 du conteneur 10 est remplacée par une bride semi-rigide 14b. Cette bride 14b porte l'obturateur 16. Elle présente sur sa surface extérieure une partie en saillie, apte à venir s'emboîter à force dans une partie en creux formée à l'intérieur d'une bride 30a montée à l'extrémité correspondante du tube transparent 30 du sas 28.

Toutes les autres caractéristiques du dispositif et de son procédé de mise en oeuvre restent identiques à celles qui ont été décrites précédemment en se référant à la figure 1.

Bien entendu, l'invention n'est pas limitée aux modes de réalisation qui viennent d'être décrits à titre d'exemples, mais en couvre toutes les variantes.

## Revendications

1. Procédé de transfert de produits stériles, entre un conteneur de transport (10) et un isolateur stérile (12), selon lequel :
le conteneur (10) est mis en position d'accostage par rapport à l'isolateur (12) ;
le volume clos (40) délimité entre des obturateurs respectifs (16;24,38) du conteneur (10) et de l'isolateur (12) est stérilisé; et
lesdits obturateurs (16;24,38) sont ouverts avant de procéder au transfert des produits;
**caractérisé en ce que** en position d'accostage par rapport à l'isolateur le conteneur accoste (10) sur un sas (28) raccordé extérieurement à l'isolateur (12) et normalement séparé de celui-ci par son obturateur (24,38), ledit sas (28) incluant des moyens de stérilisation (32) et délimitant le volume clos (40) ;
et caractérisé en ce le volume clos (40) est stérilisé en mettant en oeuvre des moyens de stérilisation (32) en lumière pulsée placés autour d'un tube transparent (30) délimitant ledit volume clos.

2. Procédé selon la revendication 1, dans lequel, avant d'accoster le conteneur (10) sur le sas (28), celui-ci, est raccordé sur l'isolateur (12) par l'intermédiaire d'un dispositif de transfert étanche à double porte, dont la double porte (24,38) forme l'obturateur de l'isolateur (12).

3. Procédé selon l'une quelconque des revendications précédentes, dans lequel le transfert des produits est effectué après avoir retourné à l'intérieur du sas (28) une manche de protection (18) interne au conteneur (10).

4. Procédé selon l'une quelconque des revendications précédentes, dans lequel l'obturateur (16) du conteneur (10) est ouvert par découpage depuis l'intérieur de l'isolateur (12), après ouverture de l'obturateur (24,38) de celui-ci.

5. Procédé selon l'une quelconque des revendications précédentes, dans lequel est utilisé un conteneur (10) muni d'un obturateur (16) dont la face extérieure, apte à être tournée vers le volume clos (40), est réfléchissante.

6. Procédé selon l'une quelconque des revendications précédentes, dans lequel un conteneur (10) souple est utlisé et celui-ci est accosté sur le sas (28) par emmanchement élastique.

7. Procédé selon l'une quelconque des revendications précédentes, dans lequel est utilisé un conteneur (10) présentant une bride semi-rigide (14b) portant l'obturateur (16) du conteneur (10), et celui-ci est accosté sur le sas (28) par emboîtement de la bride (14b) dans une bride (30a) complémentaire du sas.

8. Dispositif de transfert de produits stériles entre un conteneur de transport (10) et un isolateur stérile (12), comprenant :
- un obturateur (16) de conteneur ;
- un obturateur (24,38) d'isolateur ;
- des moyens d'accostage du conteneur (10) sur l'isolateur (12), aptes à délimiter un volume clos (40) entre l'obturateur (16) de conteneur et l'obturateur (24,38) d'isolateur, lorsque l'accostage est réalisé ;
- des moyens de stérilisation (32) dudit volume clos (40) ;
le dispositif étant **caractérisé en ce qu'**il comprend de plus un sas (28) raccordé extérieurement à l'isolateur (12) et normalement séparé de celui-ci par l'obturateur (24,38) d'isolateur, ledit sas incluant les moyens de stérilisation (32) et comprenant un tube transparent (30) délimitant le volume clos (40) entre les obturateurs (16 ; ; 24, 38) de conteneur et d'isolateur, les moyens de stérilisation (32) étant des moyens de stérilisation en lumière pulsée et entourant le tube transparent (30).

9. Dispositif selon la revendication 8, dans lequel l'obturateur d'isolateur comprend une double porte (24,38) d'un dispositif de transfert étanche à double porte interposé entre le sas (28) et l'isolateur (12).

10. Dispositif selon l'une quelconque des revendications 8 et 9, comprenant de plus une manche de protection (18) interne au conteneur (10), apte à être retournée à l'intérieur du sas (28) lorsque l'accostage est réalisé et les obturateurs (16;24,38) de conteneur et d'isolateur ouverts.

11. Dispositif selon l'une quelconque des revendications 8 à 10, dans lequel l'obturateur (16) de conteneur est apte à être découpé depuis l'intérieur de l'isolateur (12).

12. Dispositif selon l'une quelconque des revendications 8 à 11, dans lequel l'obturateur (16) de conteneur présente une face extérieure réfléchissante, prévue pour être tournée vers le volume clos (40).

13. Dispositif selon l'une quelconque des revendications 8 à 12, dans lequel le conteneur (10) présente une paroi souple (14), les moyens d'accostage incluant une partie élastique (14a) de la paroi, entourant l'obturateur (16) de conteneur et apte à être emmanchée sur le sas (28).

14. Dispositif selon l'une quelconque des revendications 8 à 12, dans lequel le conteneur (10) présente une bride semi-rigide (14b) portant l'obturateur (16) de conteneur et le sas (28) présente une bride (30a) complémentaire de la bride du conteneur, et dans laquelle celle-ci peut être emboîtée, pour former les moyens d'accostage.

## Patentansprüche

1. Verfahren zum Transport steriler Produkte zwischen einem Transportbehälter (10) und einem sterilen Isolierelement (12), gemäß dem:
der Behälter (10) in bezug auf das Isolierelement (12) in eine Anlageposition gebracht wird,
das geschlossene Volumen (40), das zwischen betreffenden Verschlüssen (16;24,38) des Behälters (10) und dem Isolierelement (12) festgelegt ist, sterilisiert wird, und
die Verschlüsse (16;24,38) geöffnet werden, bevor der Transport der Produkte durchgeführt wird,
**dadurch gekennzeichnet, daß** der in der Anlageposition in bezug auf das Isolierelement befindliche Behälter (10) an eine Schleuse (28) angelegt wird, die außen an dem Isolierelement (12) angeschlossen und normalerweise von diesem durch dessen Verschluß (24,38) getrennt ist; wobei die Schleuse (28) Sterilisierungsmittel (32) aufweist und das geschlossene Volumen (40) festlegt, und
**dadurch gekennzeichnet, daß** das geschlossene Volumen (40) sterilisiert wird, in dem die Sterilisierungsmittel (32) die um ein lichtdurchlässiges Rohr (30) herum plaziert sind, welche das geschlossene Volumen festlegt, mit gepulstem Licht betätigt werden.

2. Verfahren nach Anspruch 1, wobei vor dem Anlegen des Behälters (10) an der Schleuse (28) diese mit dem Isolierelement (12) über eine dichte Transportvorrichtung mit Doppeltür verbunden wird, wobei die Doppeltür (24, 38) den Verschluß des Isolierelements (12) bildet.

3. Verfahren nach einem der vorangehenden Ansprüche, wobei der Transport bzw. die Übertragung der Produkte ausgeführt wird, nachdem eine im Innern des Behälters (10) befindliche Schutzhaube (18) ins Innere der Schleuse (28) zurückgezogen wurde.

4. Verfahren nach einem der vorangehenden Ansprüche, wobei der Verschluß (16) des Behälters (10) durch Schneiden bzw. Durchtrennen vom Inneren des Isolierelements (12) her nach dem Öffnen des Verschlusses (24,38) desselben geöffnet wird.

5. Verfahren nach einem der vorangehenden Ansprüche, wobei ein Behälter (10) verwendet wird, der mit einem Verschluß (16) versehen ist, dessen Außenfläche, die zu dem geschlossenen Volumen (40) hin gedreht werden kann, reflektierend ist.

6. Verfahren nach einem der vorangehenden Ansprüche, wobei ein elastischer Behälter (10) verwendet wird und dieser an der Schleuse (28) durch elastisches Einstecken angelegt wird.

7. Verfahren nach einem der vorangehenden Ansprüche, wobei ein Behälter (10) verwendet wird, der einen den Verschluß (16) des Behälters (10) tragenden halb-starren Flansch (14b) aufweist, und dieser an der Schleuse (28) durch Einpassen des Flansches (14b) in einen komplementären Flansch (30a) der Schleuse angelegt wird.

8. Vorrichtung zum Transport steriler Produkte zwischen einem Transportbehälter (10) und einem sterilen Isolierelement (12), mit
- einem Verschluß (16) des Behälters,
- einem Verschluß (24,38) des Isolierelements,
- Mitteln zum Anlegen des Behälters (10) an dem Isolierelement (12), die ein geschlossenes Volumen (40) zwischen dem Verschluß (16) des Behälters und dem Verschluß (24,38) des Isolierelements festlegen können, wenn das Anlegen bzw. Koppeln durchgeführt wird,
- Sterilisierungsmitteln (32) für das geschlossene Volumen (40), wobei die Vorrichtung
**dadurch gekennzeichnet ist, daß** sie außerdem eine äußerlich mit dem Isolierelement (12) verbundene und normalerweise von diesem durch den Verschluß (24,38) des Isolierelements getrennte Schleuse (28) umfaßt, wobei die Schleuse die Sterilisierungsmittel (32) aufweist und ein lichtdurchlässiges Rohr (30) umfaßt, welches das geschlossene Volumen (40) zwischen den Verschlüssen (16;24,38) des Behälters und des Isolierelements festlegt, wobei die Sterilisierungsmittel (32) Sterilisierungsmittel mit gepulstem Licht, welche das lichtdurchlässige Rohr (30) umgeben, sind.

9. Vorrichtung nach Anspruch 8, wobei der Verschluß des Isolierelements eine Doppeltür (24,38) einer dichten Transportvorrichtung mit Doppeltür umfaßt, die zwischen die Schleuse (28) und das Isolierelement (12) eingefügt ist.

10. Vorrichtung nach einem der Ansprüche 8 und 9, außerdem mit einer Schutzhaube (18) innerhalb des Behälters (10), die ins Innere der Schleuse (28) zurückgezogen werden kann, wenn das Anlegen bzw. Koppeln ausgeführt wird bzw. ist und die Verschlüsse (16;24,38) des Behälters bzw. des Isolierelements offen sind.

11. Vorrichtung nach einem der Ansprüche 8 bis 10, wobei der Verschluß (16) des Behälters vom Innern des Isolierelements (12) aus durchtrennt werden kann.

12. Vorrichtung nach einem der Ansprüche 8 bis 11, wobei der Verschluß (16) des Behälters eine reflektierende Außenfläche aufweist, die vorgesehen ist, um zu dem geschlossenen Volumen (40) hin gedreht zu werden.

13. Vorrichtung nach einem der Ansprüche 8 bis 12, wobei der Behälter (10) eine elastische Wand (14) aufweist, die Anlage- bzw. Kopplungsmittel einen elastischen Abschnitt (14a) der Wand umfassen, welche den Verschluß (16) des Behälters umgeben und auf die Schleuse (28) aufgesteckt werden können.

14. Vorrichtung nach einem der Ansprüche 8 bis 12, wobei der Behälter (10) einen halb-starren Flansch (14b) aufweist, der den Verschluß (16) des Behälters trägt, und die Schleuse (28) einen zum Flansch des Behälters komplementären Flansch (30a) aufweist, in den dieser eingesetzt werden kann, um die Anlage- bzw. Kopplungsmittel zu bilden.

## Claims

1. Process for transferring sterile products between a transport container (10) and a sterile isolator (12), in which:
- the container (10) is brought into the docking position relative to the isolator (12);
- the enclosed volume (40) between closers (16; 24, 38) on the container (10) and the isolator (12) is sterilized; and
- the said closers (16; 24, 38) are opened before starting to transfer the products;
**characterized in that** in the docking position relative to the isolator, the container (10) is docked onto a lock (28) connected on the outside to the isolator (12) and normally separated from it by its closer (24, 38), the said lock (28) including means of sterilization (32) and delimiting the enclosed volume (40), and **characterized in that** the enclosed volume (40) is sterilized using pulsed light sterilization means (32) placed around a transparent tube (30) delimiting the said enclosed volume.

2. Process according to claim 1, in which before the container (10) is docked onto the lock (28), the lock is connected onto the isolator (12) by means of a sealed double door transfer device, in which the double door (24, 38) forms the closer of the isolator (12).

3. Process according to any of the previous claims, in which the products are transferred after turning a protective sleeve (18) internal to the container (10), over inside the lock (28).

4. Process according to any one of the previous claims, in which the closer (16) of the container (10) is opened by cutting out from inside the isolator (12), after opening the isolator closer (24, 38).

5. Process according to any one of the previous claims, in which the container used (10) is equipped with a closer (16), in which the outside face that can face enclosed volume (40) is reflecting.

6. Process according to any one of the previous claims, in which a flexible container (10) is used and this container is docked onto the lock (28) by an elastic sleeve fitting.

7. Process according to any one of the previous claims, in which a container (10) is used with a semi-rigid flange (14b) supporting the closer (16) of the container (10), and the container is docked onto the lock (28) by fitting the flange (14b) into a complementary flange (38) on the lock.

8. Device for transferring sterile products between a transport container (10) and a sterile isolator (12) comprising:
- a container closer (16);
- an isolator closer (24, 38);
- means of docking the container (10) onto the isolator (12), in order to delimit an enclosed volume (40) between the container closer (16) and the isolator closer (24, 38) when docking is complete;
- means of sterilizing (32) the said enclosed volume (40);
the device being **characterized in that** it also includes a lock (28) connected on the outside to the isolator (12) and normally separated from the isolator by the isolator closer (24, 38), the said lock including sterilization means (32) and delimiting the enclosed volume (40), comprising a transparent tube (30) delimiting the enclosed volume (40) between the container and isolator closers (16; 24, 38), the sterilization means (32) being used consisting of pulsed light sterilization means surrounding the transparent tube (30).

9. Device according to claim 8, in which the isolator closer comprises a double door (24, 38) of a sealed double door transfer device inserted between the lock (28) and the isolator (12).

10. Device according to either of claims 8 and 9, also comprising a protective sleeve (18) internal to the container (10) that can be turned over inside the lock (28) when docking is complete, and the container and isolator closers (16; 24, 38) are open.

11. Device according to any one of claims 8 to 10, in which the container closer (16) can be cut out from inside the isolator (12).

12. Device according to any one of claims 8 to 11, in which the container closer (16) has a reflecting outside surface designed to face enclosed volume (40).

13. Device according to any one of claims 8 to 12, in which the container (10) has a flexible wall (14), the docking means including an elastic part (14a) of the wall, surrounding the container closer (16) and that can be fitted like a sleeve onto the lock (28).

14. Device according to any one of claims 8 to 12, in which the container (10) has a semi-rigid flange (14b) on which the container closer (16) is fitted, and the lock (28) has a flange (30a) complementary to the container flange, and in which the container flange may be inserted to form docking means.
